(19) Europäisches Patentamt
European Patent Office
Office européen des brevets

(11) Veröffentlichungsnummer: **0 385 938 A1**

# (12) EUROPÄISCHE PATENTANMELDUNG

(21) Anmeldenummer: **90810123.1**

(22) Anmeldetag: **20.02.90**

(51) Int. Cl.5: **C09B 29/039, C07D 333/70, C07D 513/04, //(C07D513/04, 333:00,275:00),D06P1:16, D06P1:39**

(30) Priorität: **01.03.89 CH 742/89**

(43) Veröffentlichungstag der Anmeldung: **05.09.90 Patentblatt 90/36**

(84) Benannte Vertragsstaaten: **BE CH DE ES FR GB IT LI**

(71) Anmelder: **CIBA-GEIGY AG**
**Klybeckstrasse 141**
**CH-4002 Basel(CH)**

(72) Erfinder: **Naef, Rudolf, Dr.**
**Im Budler 6**
**CH-4419 Lupsingen(CH)**

## (54) Heteroaromatische Azofarbstoffe.

(57) Farbstoffe der Formel

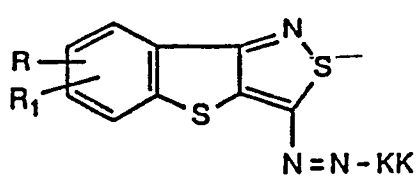

worin R und $R_1$ unabhängig voneinander je Wasserstoff, Halogen, Nitro, Alkyl oder Alkoxy und KK den Rest einer Kupplungskomponente bedeuten.

EP 0 385 938 A1

## Heteroaromatische Azofarbstoffe

Die vorliegende Eriindung betrifft Azofarbstoffe, welche sich von 3-Aminobenzothienoisothiazol als Diazokomponente ableiten, die Herstellung dieser Azofarbstoffe sowie ihre Verwendung als Farbstoffe far Textilmaterial.

Die erfindungsgemässen Farbstoffe entsprechen der Formel

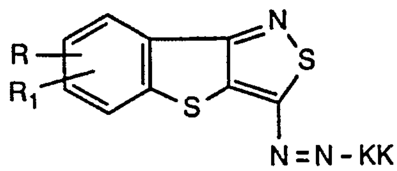

(1)

worin R und $R_1$ unabhängig voneinander je Wasserstoff, Halogen, Nitro, Alkyl oder Alkoxy und KK den Rest einer Kupplungskomponente bedeuten.

Halogen bedeutet Fluor, Brom, Jod oder vor allem Chlor.

Unter Alkylresten sind erfindungsgemäss generell geradkettige, verzweigte oder cyclische Alkylgruppen zu verstehen. Es handelt sich z.B. um Methyl, Ethyl, Propyl, i-Propyl, Butyl, i-Butyl, tert.-Butyl, Amyl, tert.-Amyl (1,1-Dimethylpropyl), 1,1,3,3-Tetramethylbutyl, Hexyl, 1-Methylpentyl, Neopentyl, 1-,2- oder 3-Methylhexyl, Heptyl, n-Octyl, tert.-Octyl, 2-Ethylhexyl, n-Nonyl, Isononyl, Decyl, Dodecyl, Cyclopentyl, Cyclohexyl, Methylcyclohexyl sowie die dazugehörenden Isomeren. Die Alkylreste enthalten vorzugsweise 1 bis 6 C-Atome, vor allem 1 bis 4 C-Atome.

Diese Alkylreste können substituiert sein, z.B. durch Hydroxy, Alkoxy, Cyan oder Phenyl. Beispiele für solche substituierten Alkylreste sind Hydroxyethyl, Methoxymethyl, Ethoxyethyl, Cyanethyl, Propoxypropyl oder Benzyl.

Geeignete Alkoxyreste sind vorzugsweise solche mit 1-4 C-Atomen z.B. Methoxy, Ethoxy, Propoxy, iso-Propoxy, n-Butoxy, iso-Butoxy oder tert.-Butoxy.

R und $R_1$ stellen vorzugsweise unabhängig voneinander je Wasserstoff, Chlor, Nitro, Methyl oder Methoxy dar.

Vorzugsweise ist $R_1$ Wasserstoff und R Wasserstoff oder Chlor.

Als Kupplungskomponente KK kommen die bei Azofarbstoffen üblichen und aus der einschlägigen Literatur bekannten Kupplungskomponenten in Frage.

Aus der Vielzahl der Möglichkeiten seien beispielsweise erwähnt: Kupplungskomponenten der Benzolreihe, der Naphthalinreihe, der offenkettigen methylenaktiven Verbindungen (wie z.B. der Acylacetarylamide) sowie der heterocyclischen Reihe.

Beispiele für die genannten Reste von Kupplungskomponenten KK sind Reste aus der Reihe der Acylacetarylamide, Phenole, Pyridone, Chinolone, Pyrazole, Indole, Diphenylamine, Aniline, Aminopyridine, Pyrimidine, Pyrimidone, Naphthole, Naphthylamine, Aminothiazole, Thiophene oder Hydroxypyridine.

Besonders zu erwähnende Reste KK sind solche aus der Reihe der Acetoacetanilide, Phenole, Aniline, Diphenylamine, Naphthylamine, Naphthole, Indole, Chinoline, Pyridone, Pyrazole, Chinolone und Aminopyridine.

Diese Kupplungskomponenten können weitere Substituenten tragen, die in der Farbstoffchemie für Kupplungskomponenten üblich sind, beispielsweise Hydroxy, Amino, Alkylamino, Dialkylamino, Halogen, Alkoxy, Aryl, Aryloxy, Alkylcarbonylamino, Arylcarbonylamino, Alkylsulfonylamino oder Sulfo.

Wegen ihrer besonders guten färberischen Eigenschaften sind solche Farbstoffe der Formel (1) besonders bevorzugt, bei denen KK den Rest eines Anilins, Naphthylamins oder Tetrahydrochinolins bedeutet, wobei diese Reste durch $C_1$-$C_4$-Alkyl, $C_1$-$C_4$-Alkylcarbonylamino, Phenyl, $C_1$-$C_4$-Alkylphenyl oder Sulfo substituiert sein können.

Besonders bevorzugte erfindungsgemässe Azofarbstoffe sind diejenigen der Formel (1), worin R Wasserstoff, Chlor, Nitro, Methyl oder Methoxy und KK den Rest einer Kupplungskomponente aus der Reihe der Acetoacetanilide, Phenole, Aniline, Diphenylamine, Naphthylamine, Naphthole, Indole, Chinoline, Pyridone, Pyrazole, Chinolone und Aminopyridine bedeutet, wobei diese Reste unsubstituiert oder durch Hydroxy, Amino, Alkyl amino, Dialkylamino, Halogen, Alkoxy, Aryl, Aryloxy, Alkylcarbonylamino, Arylcarbonylamino, Alkylsulfonylamino oder Sulfo substituiert sind. Aryl bedeutet dabei z.B. Naphthyl oder vor allem Phenyl.

Unter diesen sind diejenigen besonders bevorzugt, bei denen R Wasserstoff oder Chlor und KK den

Rest eines Anilins, Naphthylamins oder Tetrahydrochinolins bedeutet, wobei diese Reste durch $C_1$-$C_4$-Alkyl, $C_1$-$C_4$-Alkylcarbonylamino, Phenyl, $C_1$-$C_4$-Alkylphenyl oder Sulfo substituiert sein können.

Die neuen Azofarbstoffe der Formel (1) können nach an sich bekannten Verfahren hergestellt werden. Man erhält sie beispielsweise, indem man eine Verbindung der Formel

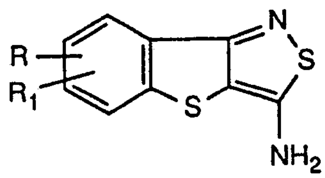

(2)

diazotiert und auf eine Kupplungskomponente

H-KK (3)

kuppelt, wobei R, $R_1$ und KK die unter der Formel (1) angegebene Bedeutung aufweisen.

Die Diazotierung der Verbindungen der Formel (2) erfolgt in an sich bekannter Weise, z.B. mit Natriumnitrit in saurem, z.B. salzsaurem oder schwefelsaurem, wässrigem Medium. Die Diazotierung kann aber auch mit anderen Diazotierungsmitteln, z.B. mit Nitrosylschwefelsäure ausgeführt werden. Bei der Diazotierung kann eine zusätzliche Säure im Reaktionsmedium anwesend sein, z.B. Phosphorsäure, Schwefelsäure, Essigsäure, Propionsäure, Salzsäure oder Mischungen dieser Säuren, z.B. Mischungen aus Phosphorsäure und Essigsäure. Zweckmässig wird die Diazotierung bei Temperaturen von -10 bis 30 °C, z.B. von -10 °C bis Raumtemperatur, durchgeführt.

Die Kupplung der diazotierten Verbindung der Formel (2) auf die Kupplungskomponente H-KK erfolgt ebenfalls in bekannter Weise, beispielsweise in saurem, wässrigem oder wässrig-organischem Medium, vorteilhaft bei Temperaturen von -10 bis 30 °C, insbesondere unter 10 °C. Als Säuren verwendet man z.B. Salzsäure, Essigsäure, Schwefelsäure oder Phosphorsäure. Diazotierung und Kupplung können beispielsweise im Eintopfverfahren, also im gleichen Reaktionsmedium durchgeführt werden.

Die Kupplungskomponenten H-KK sind bekannt.

Die Verbindungen der Formel

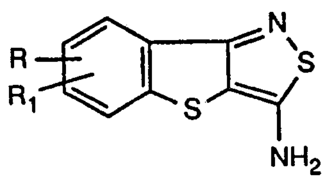

(2)

worin R und $R_1$ je Wasserstoff, Halogen, Nitro, Alkyl oder Alkoxy bedeutet, sind neu und stellen einen weiteren Gegenstand der vorliegenden Erfindung dar.

Man erhält die Verbindungen der Formel (2) z.B., indem man eine Verbindung der Formel

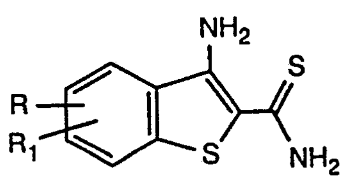

(4)

worin R und $R_1$ je Wasserstoff, Halogen, Nitro, Alkyl oder Alkoxy bedeutet, in Gegenwart einer organischen Base mit einem Oxidationsmittel behandelt. Die Reaktion wird vorzugsweise bei einer Temperatur zwischen -20 und 50 °C, vor allem zwischen -10 und 20 °C, in einem inerten Lösungsmittel, wie z.B. Dimethylformamid oder Dimethylsulfoxid durchgeführt.

Die Reaktion kann aber auch ohne inertes Lösungsmittel durchgeführt werden, wobei in diesem Falle die organische Base in solcher Menge verwendet wird, dass sie auch als Lösungsmittel dient.

Als organische Basen dienen z.B. Pyridin, $\alpha,\beta$- oder $\gamma$-Picolin oder Lutidine, z.B. 2,4-oder 2,6-Lutidin. Bevorzugt unter diesen ist Pyridin.

Als Oxidationsmittel wird vor allem Wasserstoffperoxid eingesetzt, vorzugsweise als wässrige Lösung.

Die Verbindungen der Formel

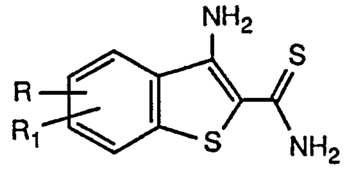

(4)

worin R und $R_1$ je Wasserstoff, Halogen, Nitro, Alkyl oder Alkoxy bedeutet, sind neu und stellen einen weiteren Gegenstand der vorliegenden Erfindung dar.

Man erhält die Verbindungen der Formel (4) z.B., indem man in Verbindungen der Formel

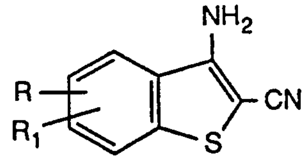

(5)

worin R und $R_1$ je Wasserstoff, Halogen, Nitro, Alkyl oder Alkoxy bedeutet, die Nitrilgruppe in an sich bekannter Weise in die Thioamidgruppe umwandelt, z.B. durch Erhitzen einer Verbindung der Formel (5) zusammen mit Phosphorpentasulfid auf ca. 120 °C, durch Erhitzen einer ethanolischen Lösung einer Verbindung der Formel (5) zusammen mit Schwefel unter Rückfluss, durch Erhitzen einer Verbindung der Formel (5) zusammen mit $H_2S$ in flüssigem Triethylamin im Autoklav auf ca. 50 °C oder vorzugsweise durch Einleiten von $H_2S$ bei ca. 30 bis 70 °C in eine Lösung einer Verbindung der Formel (5) in einem inerten Lösungsmittel, wie z.B. Dimethylsulfoxid.

Die Verbindungen der Formel (5) sind bekannt oder können auf bekannte Art hergestellt werden, z.B. nach der in J. Org. Chem. 39,3440(1974) beschriebenen Arbeitsweise.

Falls die erfindungsgemässen Verbindungen der Formel (1) keine wasserlöslichmachenden Gruppen, insbesondere keine Sulfogruppen aufweisen, so können sie als Dispersionsfarbstoffe zum Färben und Bedrucken von halbsynthetischen und insbesondere synthetischen hydrophoben Fasermaterialien, vor allem Textilmaterialien, verwendet werden. Textilmaterialien aus Mischgeweben, die derartige halbsynthetische bzw. synthetische hydrophobe Textilmaterialien enthalten, können ebenfalls mit Hilfe der erfindungsgemässen Verbindungen gefärbt oder bedruckt werden.

Als halbsynthetische Textilmaterialien kommen vor allem Cellulose-2 1/2-Acetat und Cellulosetriacetat in Frage.

Synthetische hydrophobe Textilmaterialien bestehen vor allem aus linearen, aromatischen Polyestern, beispielsweise solchen aus Terephthalsäure und Glykolen, besonders Ethylenglykol oder Kondensationsprodukten aus Terephthalsäure und 1,4-Bis-(hydroxymethyl)-cyclohexan; aus Polycarbonaten, z.B. solchen aus $\alpha,\alpha$-Dimethyl-4,4′-dihydroxy-diphenylmethan und Phosgen, aus Fasern auf Polyvinylchlorid- sowie Polyamid-Basis.

Die Applikation der erfindungsgemässen Dispersionsfarbstoffe auf die Textilmaterialien erfolgt nach bekannten Färbeverfahren. Beispielsweise färbt man Polyesterfasermaterialien im Ausziehverfahren aus wässriger Dispersion in Gegenwart von üblichen anionischen oder nicht-ionischen Dispergiermitteln und gegebenenfalls üblichen Quellmitteln (Carrier) bei Temperaturen zwischen 80 und 140 °C. Cellulose-2 1/2-acetat färbt man vorzugsweise zwischen ungefähr 65 bis 85 °C und Cellulosetriacetat bei Temperaturen bis zu 115 °C.

Die neuen Dispersionsfarbstoffe färben im Färbebad gleichzeitig anwesende Wolle und Baumwolle nicht oder nur wenig an (sehr gute Reserve), so dass sie auch gut zum Färben von Polyester/Wolle- und Polyester/Cellulosefaser-Mischgeweben verwendet werden können.

Die erfindungsgemässen Dispersionsfarbstoffe eignen sich aber insbesonders zum Färben nach dem Thermosol-Verfahren.

Das genannte Textilmaterial kann dabei in den verschiedensten Verarbeitungsformen vorliegen, wie z.B. als Faser, Faden oder Vlies, als Gewebe oder Gewirke.

Es ist vorteilhaft, die erfindungsgemässen Dispersionsfarbstoffe vor ihrer Verwendung in ein Farbstoffpräparat überzuführen. Hierzu wird der Farbstoff vermahlen, so dass seine Teilchengrösse im Mittel zwischen 0,01 und 10 Mikron beträgt. Das Vermahlen kann in Gegenwart von Dispergiermitteln erfolgen. Beispielsweise wird der getrocknete Farbstoff mit einem Dispergiermittel gemahlen oder in Pastenform mit einem Dispergiermittel geknetet und hierauf im Vakuum oder durch Zerstäuben getrocknet. Mit den so

erhaltenen Präparaten kann man nach Zugabe von Wasser färben und bedrucken.

Beim Bedrucken wird man die üblichen Verdickungsmittel verwenden, z.B. modifizierte oder nichtmodifizierte natürliche Produkte, beispielsweise Alginate, British-Gummi, Gummi arabicum, Kristallgummi, Johannisbrotkernmehl, Tragant, Carboxymethylcellulose, Hydroxyethylcellulose, Stärke oder synthetische Produkte, beispielsweise Polyacrylamide, Polyacrylsäure oder deren Copolymere oder Poylvinylalkohole.

Die erfindungsgemässen Dispersionsfarbstoffe sind praktisch unempfindlich gegen Carrier und verleihen den genannten Materialien, vor allem dem Polyestermaterial, egale blaue bis violette Farbtöne von sehr guten Gebrauchs-Echtheiten, wie vor allem guter Lichtechtheit, Thermofixier-, Plissier-, Chlor- und Nassechtheit wie Wasser-, Schweiss- und Waschechtheit; die Ausfärbungen sind ferner gekennzeichnet durch eine gute pH-Stabilität und sehr gute Reibechtheit. Es werden ausserdem sehr farbstarke Färbungen erzielt. Besonders hervorzuheben sind die gute Sublimierechtheit und die gute Thermofixierechtheit der erhaltenen Färbungen.

Die erfindungsgemässen Farbstoffe können auch gut verwendet werden zur Herstellung von Mischnuancen zusammen mit anderen Farbstoffen. Selbstverständlich können auch Mischungen der erfindungsgemässen Farbstoffe untereinander verwendet werden.

Falls die Monoazofarbstoffe der Formel (1) eine Sulfogruppe enthalten, so liegt diese entweder in der Form der freien Sulfonsäure oder vorzugsweise als deren Salz vor. Als Salze kommen beispielsweise die Alkali-, Erdalkali- oder Ammoniumsalze oder die Salze eines organischen Amins in Betracht. Als Beispiele seien die Natrium-, Lithium-, Kalium-oder Ammoniumsalze oder das Salz des Triäthanolamins genannt.

Die sulfogruppenhaltigen Azofarbstoffe der Formel (1) können isoliert und zu brauchbaren, trockenen Färbepräparaten verarbeitet werden. Die Isolierung erfolgt vorzugsweise bei möglichst niedrigen Temperaturen durch Aussalzen und Filtrieren. Die filtrierten Farbstoffe können gegebenenfalls nach Zugabe von Coupagemitteln und/oder Puffermitteln, z.B. nach Zugabe eines Gemisches gleicher Teile Mono- und Dinatriumphosphat, getrocknet werden; vorzugsweise wird die Trocknung bei nicht zu hohen Temperaturen und unter vermindertem Druck vorgenommen. Durch Zerstäubungstrocknung des ganzen Herstellungsgemisches kann man in gewissen Fällen die erfindungsgemässen trockenen Präparate direkt, d.h. ohne Zwischenisolierung der Farbstoffe herstellen.

Die sulfogruppenhaltigen Azofarbstoffe der Formel (1) eignen sich zum Färben und Bedrucken der verschiedensten Materialien, insbesondere aber von Seide, Wolle und Polyamidfasern. Sie eignen sich sowohl für das Ausziehverfahren als auch zum Färben nach dem Foulardfärbeverfahren, wonach die Ware mit wässrigen und gegebenenfalls auch salzhaltigen Farbstofflösungen imprägniert wird.

Sie eignen sich auch zum Bedrucken von stickstoffhaltigen Fasern, z.B. von Wolle, Polyamiden oder von Seide oder Wolle enthaltenden Mischgeweben.

Es empfiehlt sich, die Färbungen und Drucke einem gründlichen Spülen mit kaltem und heissem Wasser, gegebenenfalls unter Zusatz eines dispergierend wirkenden und die Diffusion der nicht fixierten Anteile fördernden Mittels zu unterwerfen.

Die sulfogruppenhaltigen Azofarbstoffe der Fomel (1) ergeben Färbungen mit guten Nass-und Lichtechtheiten. Besonders hervorzuheben ist es, dass die Farbstoffe eine gute Löslichkeit und Elektrolytlöslichkeit bei guten Ausziehеigenschaften und hoher Farbstoff-Fixierung aufweisen, und dass sich die nicht fixierten Anteile leicht entfernen lassen. Ausser der guten Wasserlöslichkeit zeichnen sich die sulfogruppenhaltigen Azofarbstoffe der Formel (1) durch hohe Lösungsstabilität in Klotzflotten auch bei verschiedenen pH-Werten sowie sehr gute Druckpastenstabilität aus.

In den folgenden Beispielen stehen Teile für Gewichtsteile und % für Gewichtsprozent. Die Temperaturen sind Celsiusgrade.

Beispiel 1:

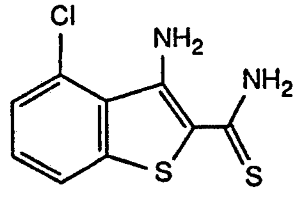

In 55 ml Dimethylsulfoxid werden 4,2 g (20 mmol) 3-Amino-4-chlor-2-cyanobenzo[b]thiophen gelöst und

bei 50°C während 24 h Schwefelwasserstoffgas eingeleitet, wobei das Produkt langsam ausfällt. Durch Zugabe von Wasser wird die Ausfällung vervollständigt. Man erhält 4,3 g Rohprodukt, welches abfiltriert wird. Nach Umkristallisation aus Dimethylformamid/Wasser bleibt ein gelbes Pulver vom Smp. 190°C zurück.

Beispiel 2:

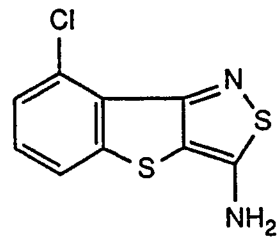

In einer Mischung von 150 ml Dimethylformamid und 50 ml Pyridin werden 36 g (148 mmol) 3-Amino-2-thioamido-4-chlorbenzo[b]thiophen gelöst und bei 0°C langsam 24,5 ml (280 mmol) 40%-ige wässrige Wasserstoffperoxid-Lösung zugetropft. Nach 24-stündigem Rühren bei Raumtemperatur wird filtriert und durch Zugabe von Wasser zur Mutterlauge 27,9 g Rohprodukt ausgefällt, das nach Umkristallisation aus Ethanol/Wasser ein gelbliches Pulver vom Smp. >250°C ergibt.

Beispiel 3:

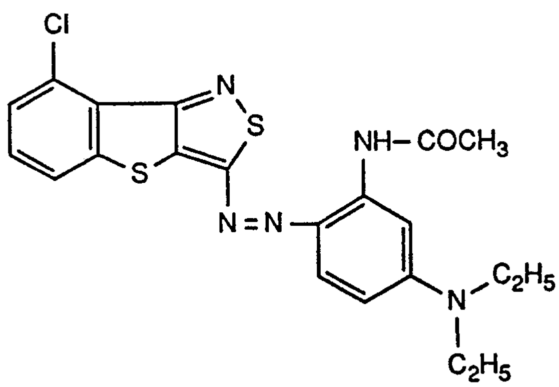

In einem Gemisch von 30 ml Essigsäure und 10 ml Propionsäure werden 1,45 g (6 mmol) 3-Amino-8-chlor-benzo[b]thieno[4,5-c]isothiazol suspendiert, nach Zugabe von 3,5 ml konz. Schwefelsäure auf 0°C abgekühlt und durch Zugabe von 0,4 g Natriumnitrit in 10 ml Wasser diazotiert. Die gelbe Diazoniumsalz-Lösung wird anschliessend zu einer Lösung von 1,5 g (6 mmol) 3-N,N-Diethylaminoacetanilid in einer Mischung aus 20 ml Essigsäure und 25 ml Ethanol getropft, wobei sofort ein rot-violetter Niederschlag ausfällt, welcher abfiltriert und mit Wasser gewaschen wird. Nach Umkristallisation aus Dimethylformamid/Wasser bleibt 1 g violettes Pulver vom Smp. >250°C zurück, welches Polyesterfasern in brillanten Violett-Tönen färbt.

Beispiele 4-7:

Auf analoge Art wie im Beispiel 3 beschrieben werden die in der folgenden Tabelle aufgeführten Azofarbstoffe erhalten, indem man die gemäss Beispiel 2 erhaltene Diazokomponente diazotiert und mit den entsprechenden Kupplungskomponenten kuppelt. In Spalte 3 der Tabelle sind die Nuancen der Färbungen auf Textilmaterial aus Polyester angegeben.

| Beispiel | Farbstoff | Nuance auf Polyester |
|---|---|---|
| 4 | 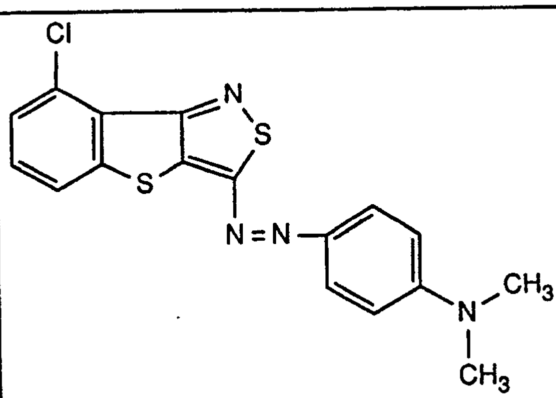 | blaustichig rot |
| 5 | 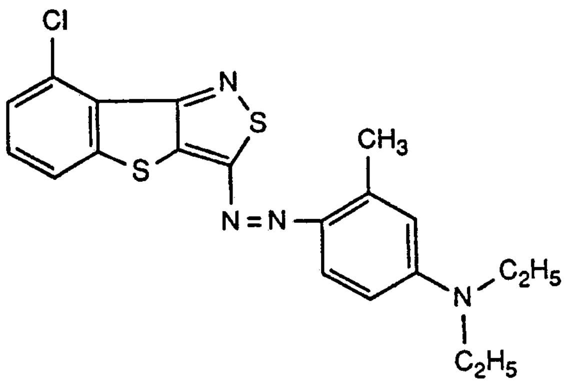 | blaustichig rot |
| 6 | 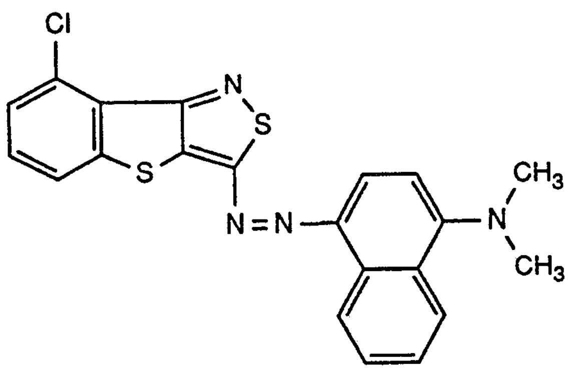 | trübes violett |
| 7 | 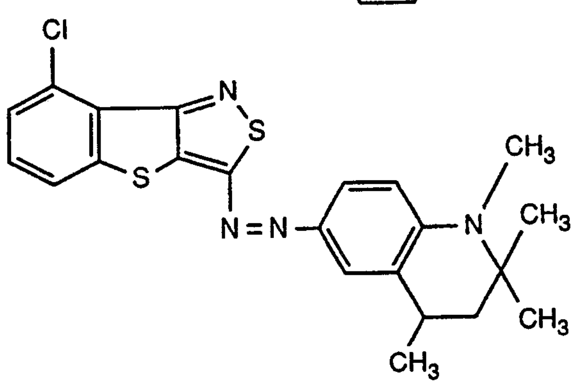 | weinrot |

Beispiel 8:

In einer Mischung von 40 ml Eisessig und 10 ml Propionsäure werden 2,4 g 3-Amino-8-chlor-benzo[b]-thieno[4,5-c]isothiazol suspendiert und nach Abkühlen auf 0° C mit 5 ml konz. Schwefelsäure versetzt. Unter guter Kühlung werden 0,7 g Natriumnitrit, gelöst in 10 ml Wasser, zugetropft, wobei sich die Suspension allmählich mit rosaroter Farbe löst. Die erhaltene Lösung wird in eine Lösung von 3,2 g 1-(4-Toluidino)-naphthalin-8-sulfonsäure in einer Mischung von 30 ml Eisessig, 40 ml Ethanol und 4 g Natriumacetat getropft, wobei die Temperatur durch Eiszugabe unter 5° C und der pH durch stetige Zugabe von 2 N Natronlauge bei ca. 3,5 gehalten wird. Der dunkelblaue schmierige Niederschlag wird abgenutscht und aus Dimethylformamid/Wasser umkristallisiert.

Der Farbstoff entspricht der Formel

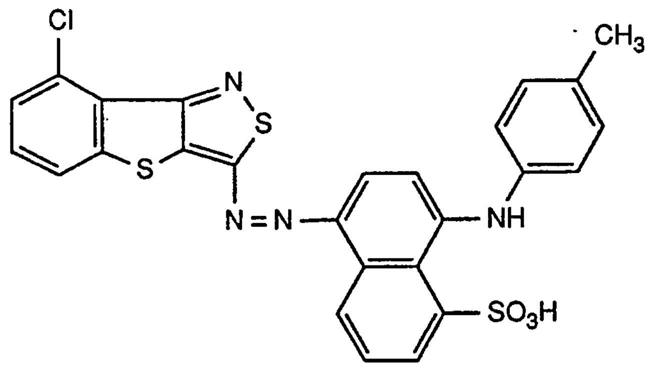

und färbt Textilmaterial aus Polyamid in brillant blauen Tönen.

Beispiele 9-10:

Auf analoge Art wie im Beispiel 8 beschrieben werden die in der folgenden Tabelle aufgeführten Azofarbstoffe erhalten, indem man die gemäss Beispiel 2 erhaltene Diazokomponente diazotiert und mit den entsprechenden Kupplungskomponenten kuppelt. In Spalte 3 der Tabelle sind die Nuancen der Färbungen auf Textilmaterial aus Polyamid angegeben.

| Beispiel | Farbstoff | Nuance auf Polamid |
|---|---|---|
| 9 | 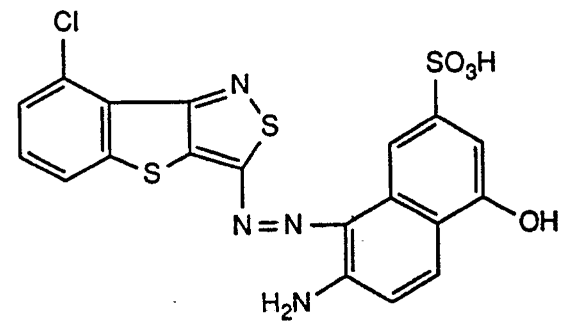 | rubinrot |
| 10 | 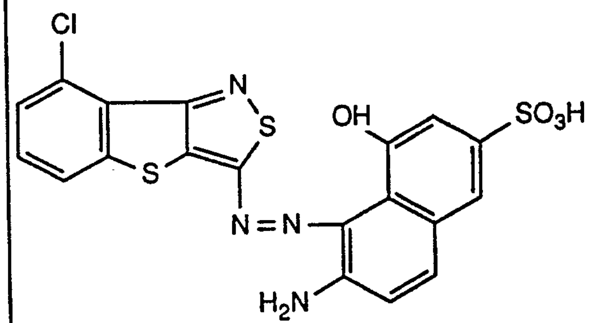 | rotstichig marineblau |

Beispiele 11-58:

Auf analoge Art wie im Beispiel 3 beschrieben werden die analogen Azofarbstoffe erhalten, indem man die in Spalte 2 der folgenden Tabelle aufgeführten Diazokomponenten diazotiert und mit den in Spalte 3 aufgeführten Kupplungskomponenten kuppelt. In Spalte 4 sind die Nuancen der Färbungen auf Textilmaterial aus Polyester angegeben.

| Beispiel | Diazokomponente | Kupplungskomponente | Nuance auf PES |
|---|---|---|---|
| 11 | 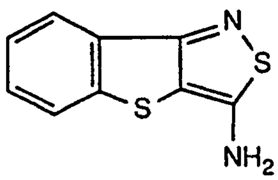 | 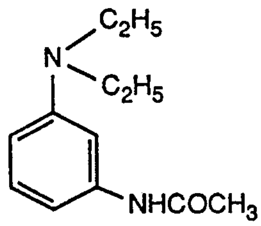 | violett |
| 12 | 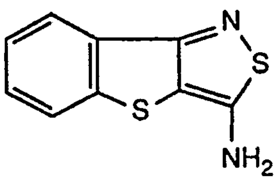 | 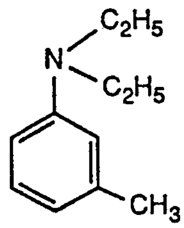 | rotstichig violett |
| 13 | 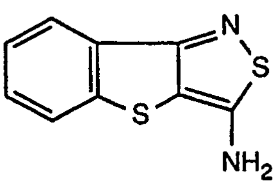 | 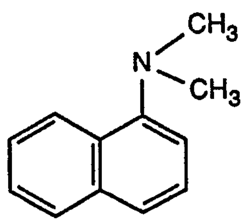 | violett |
| 14 | 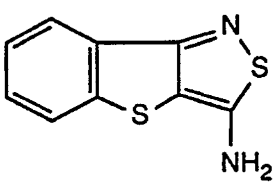 | 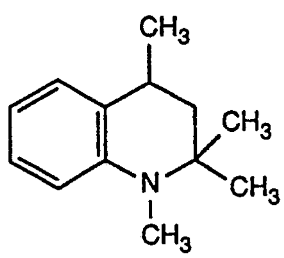 | rotstichig violett |
| 15 | 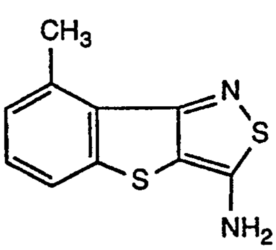 | 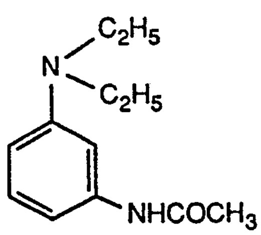 | violett |

| Beispiel | Diazokomponente | Kupplungskomponente | Nuance auf PES |
| --- | --- | --- | --- |
| 16 | 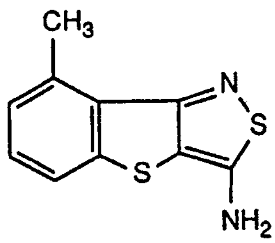 | 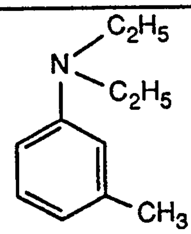 | rotstichig violett |
| 17 | 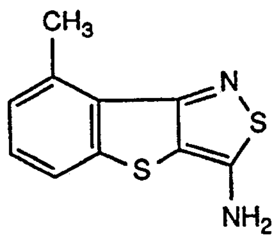 | 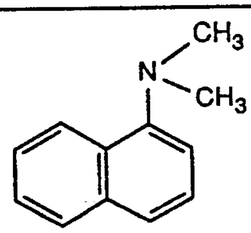 | violett |
| 18 | 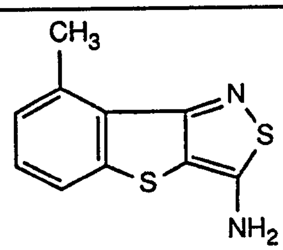 | 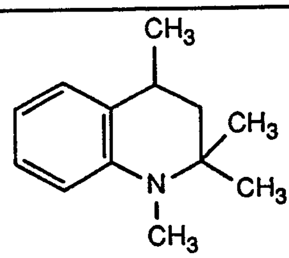 | rotstichig violett |
| 19 | 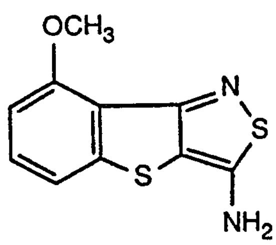 | 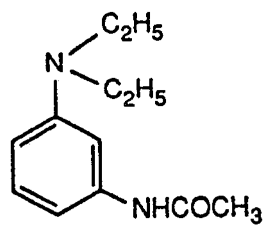 | rotstichig violett |
| 20 | 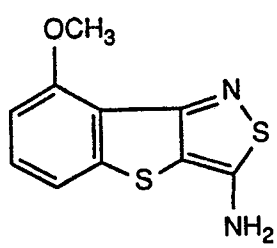 | 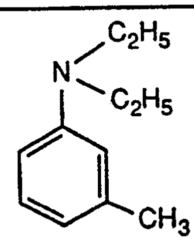 | violettstichig rot |

| Beispiel | Diazokomponente | Kupplungskomponente | Nuance auf PES |
|---|---|---|---|
| 21 | 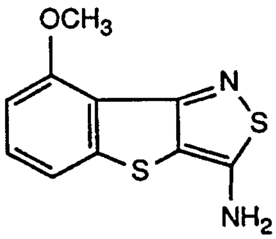 | 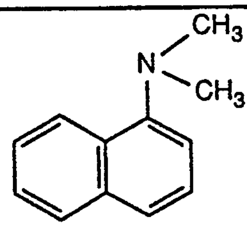 | violett |
| 22 | 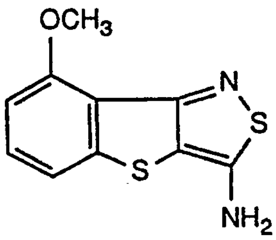 | 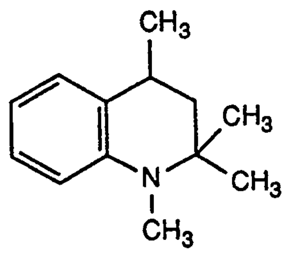 | violett |
| 23 | 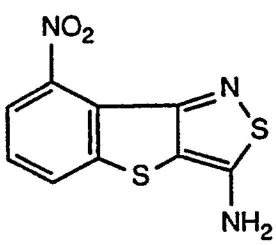 | 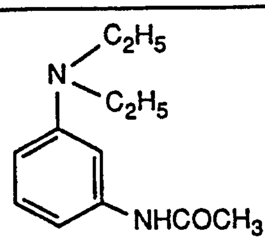 | violett |
| 24 | 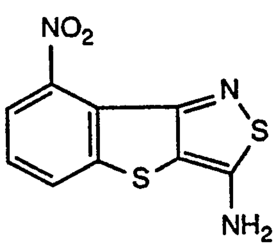 | 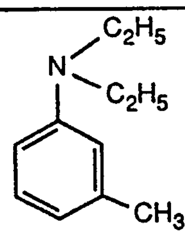 | violett |
| 25 | 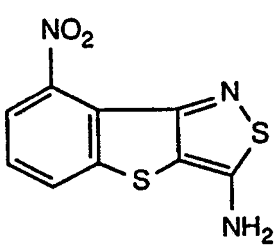 | 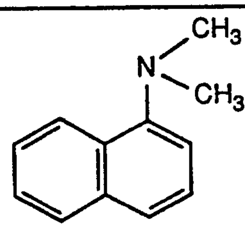 | blauviolett |

| Beispiel | Diazokomponente | Kupplungskomponente | Nuance auf PES |
|---|---|---|---|
| 26 | 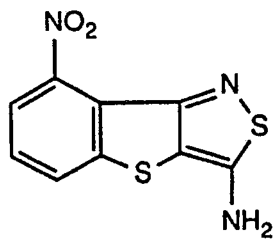 | 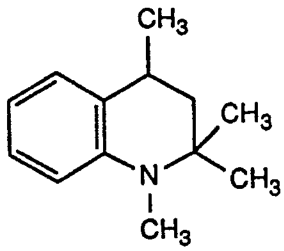 | blaustichig violett |
| 27 | 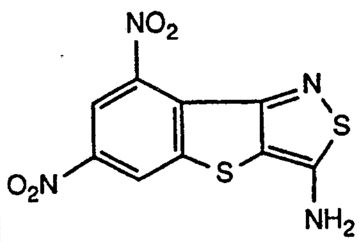 | 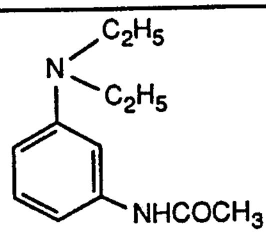 | blauviolett |
| 28 | 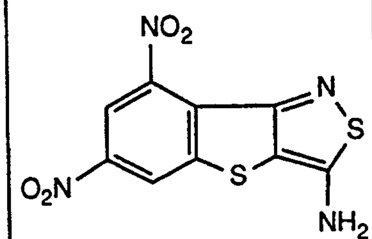 | 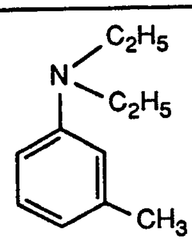 | violett |
| 29 | 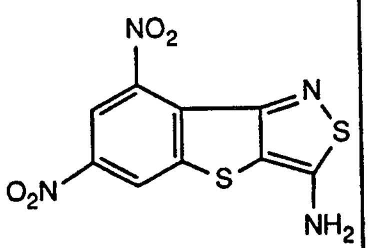 | 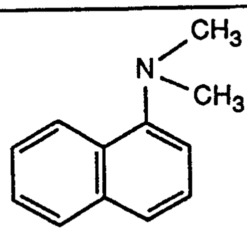 | blaustichig violett |
| 30 | 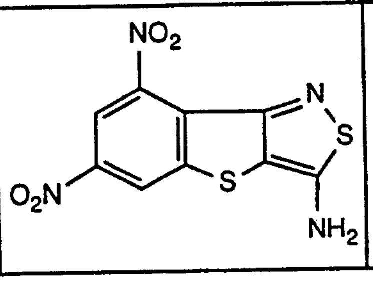 | 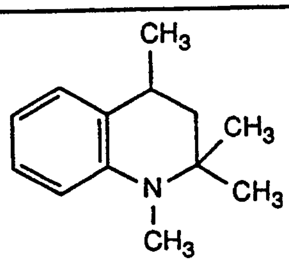 | violett |

| Beispiel | Diazokomponente | Kupplungskomponente | Nuance auf PES |
|---|---|---|---|
| 31 | 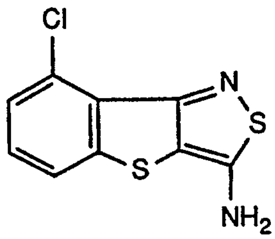 | 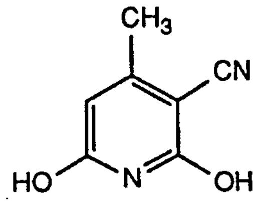 | gelb |
| 32 | 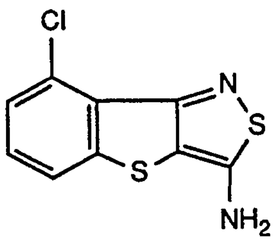 | 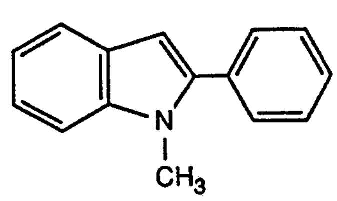 | bordeaux |
| 33 | 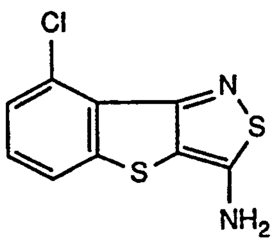 | 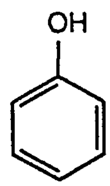 | orange |
| 34 | 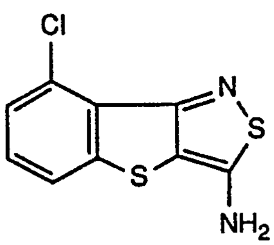 | 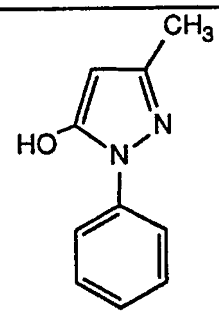 | gelbbraun |
| 35 | 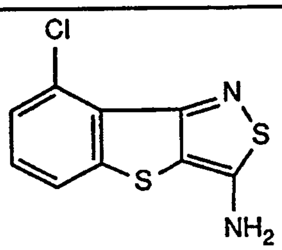 | 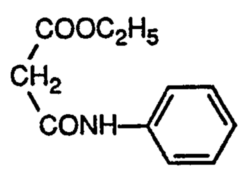 | gelb |
| 36 | 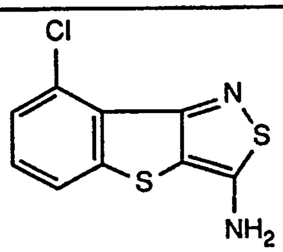 | 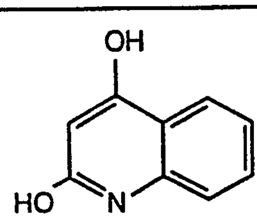 | rotstichig gelb |

| Beispiel | Diazokomponente | Kupplungskomponente | Nuance auf PES |
|---|---|---|---|
| 37 | 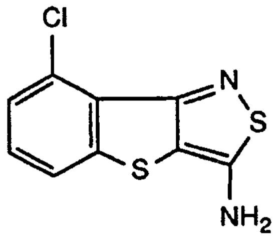 | 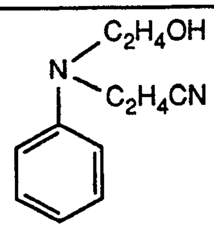 | bordeaux |
| 38 | 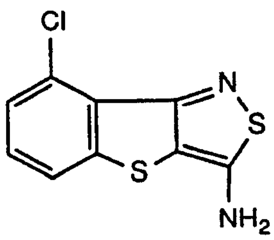 | 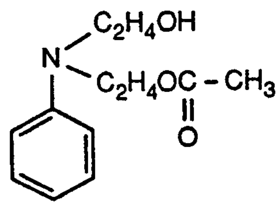 | rotviolett |
| 39 | 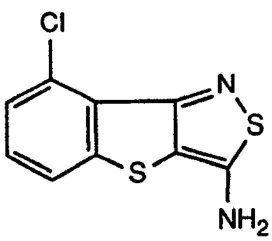 | 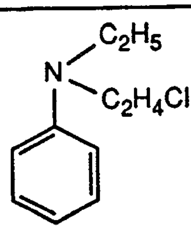 | bordeaux |
| 40 | 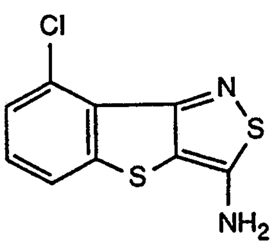 | 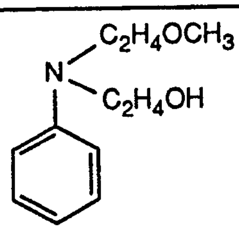 | rotviolett |
| 41 | 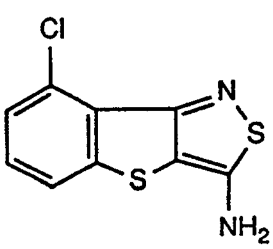 | 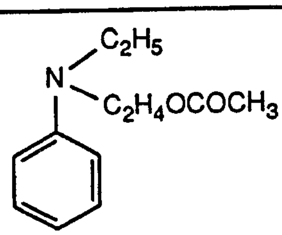 | rotviolett |

| Beispiel | Diazokomponente | Kupplungskomponente | Nuance auf PES |
|---|---|---|---|
| 42 | 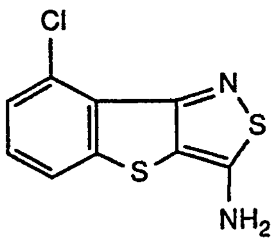 | 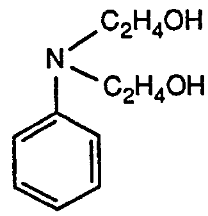 | bordeaux |
| 43 | 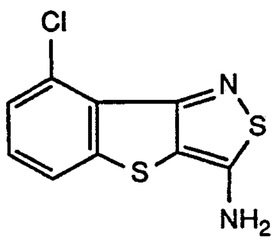 | 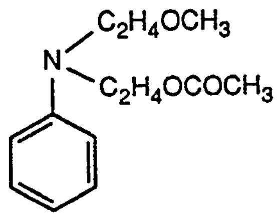 | bordeaux |
| 44 | 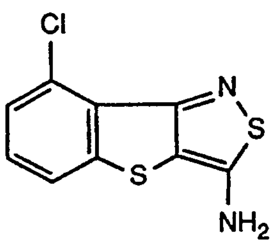 | 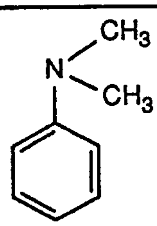 | bordeaux |
| 45 | 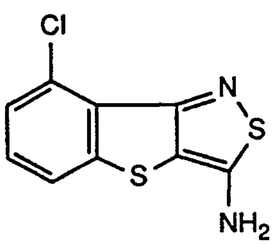 | 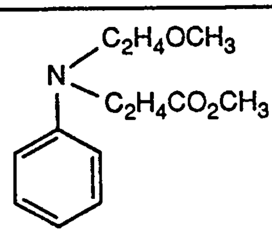 | rotviolett |
| 46 | 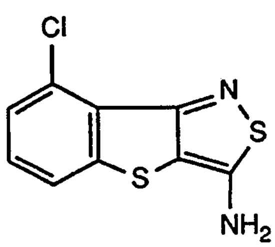 | 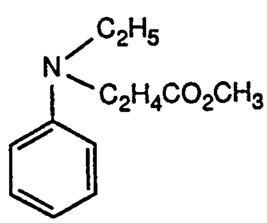 | rotviolett |

| Beispiel | Diazokomponente | Kupplungskomponente | Nuance auf PES |
|---|---|---|---|
| 47 | 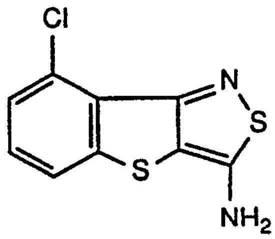 | 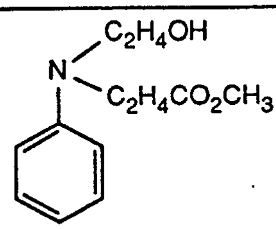 | rotviolett |
| 48 | 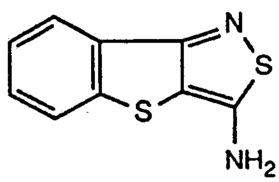 | 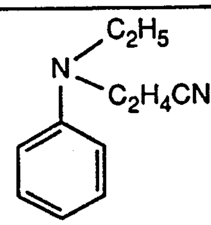 | bordeaux |
| 49 | 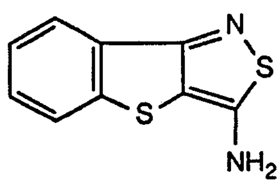 | 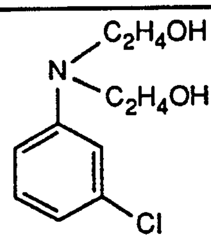 | bordeaux |
| 50 | 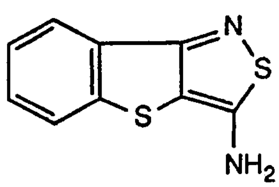 | 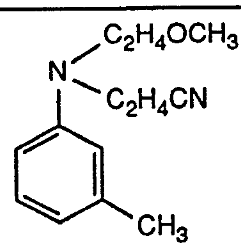 | bordeaux |
| 51 | 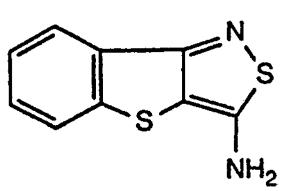 | 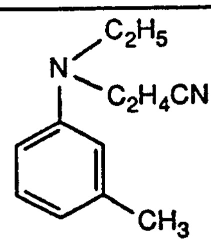 | rotviolett |

| Beispiel | Diazokomponente | Kupplungskomponente | Nuance auf PES |
|---|---|---|---|
| 52 | 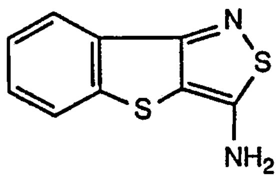 | 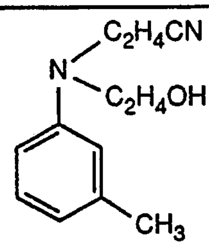 | rotviolett |
| 53 | 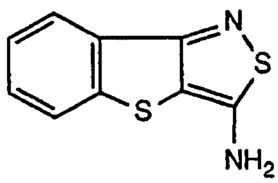 | 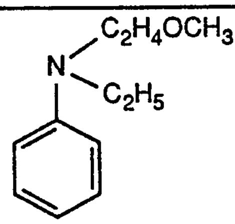 | bordeaux |
| 54 | 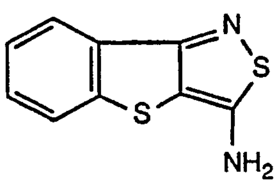 | 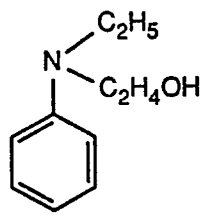 | rotviolett |
| 55 | 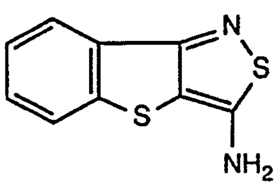 | 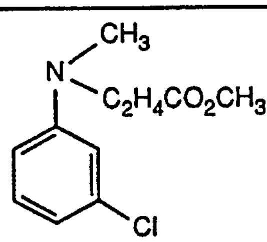 | braunrot |
| 56 | 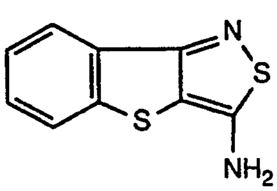 | 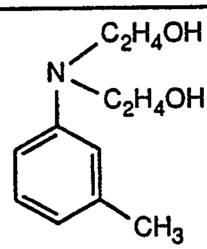 | rotviolett |

| Beispiel | Diazokomponente | Kupplungskomponente | Nuance auf PES |
|---|---|---|---|
| 57 | 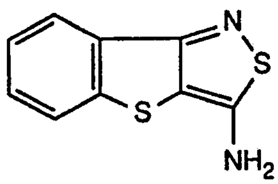 | 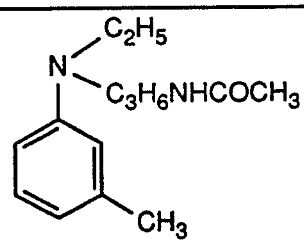 | violett |
| 58 | 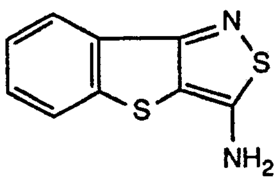 | 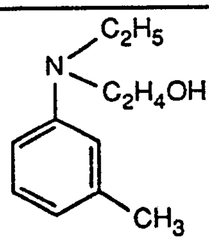 | rotviolett |

Beispiele 59-90:

Auf analoge Art wie im Beispiel 8 beschrieben werden die analogen Azofarbstoffe erhalten, indem man die in Spalte 2 der folgenden Tabelle aufgeführten Diazokomponenten diazotiert und mit den in Spalte 3 aufgeführten Kupplungskomponenten kuppelt. In Spalte 4 sind die Nuancen der Färbungen auf Textilmaterial aus Polyamid angegeben.

| Beispiel | Diazokomponente | Kupplungskomponente | Nuance auf Polyamid |
|---|---|---|---|
| 59 | 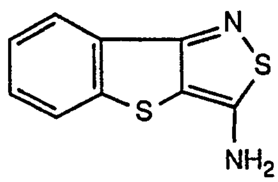 | 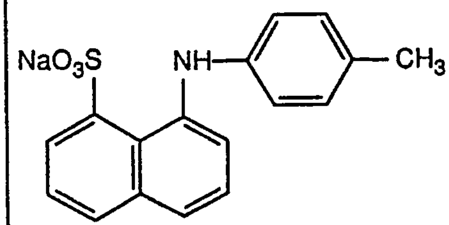 | grünstichig blau |
| 60 | 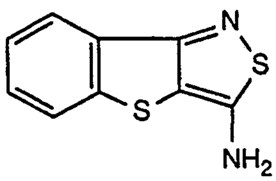 | 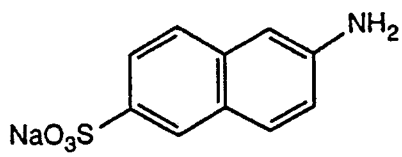 | rot |
| 61 | 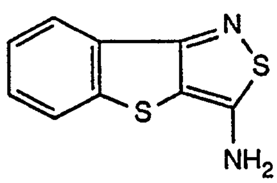 | 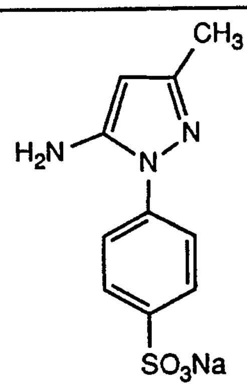 | orangegelb |
| 62 | 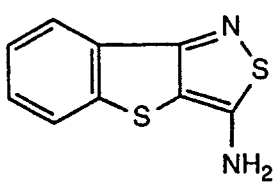 | 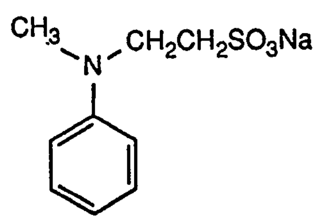 | violett |
| 63 | 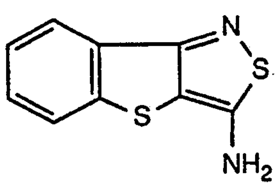 | 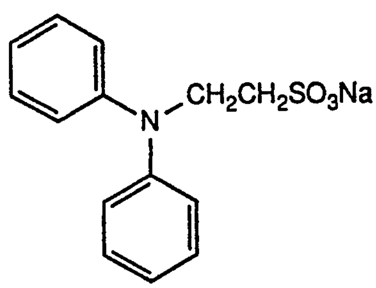 | rotviolett |

| Beispiel | Diazokomponente | Kupplungskomponente | Nuance auf Polyamid |
|---|---|---|---|
| 64 | 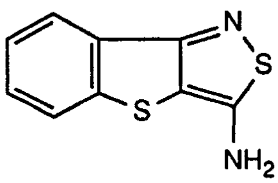 | 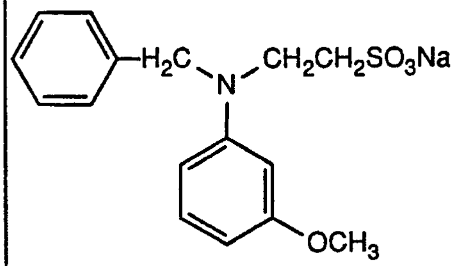 | rotstichig violett |
| 65 | 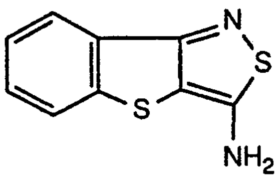 | 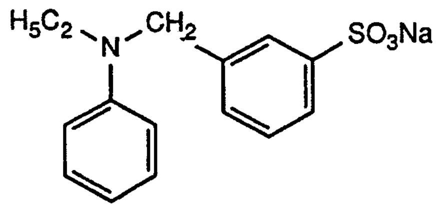 | violett |
| 66 | 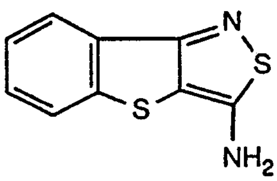 | 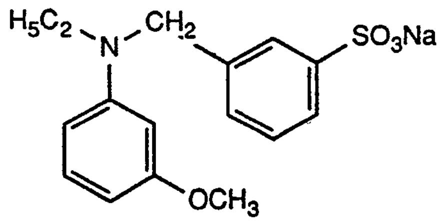 | rotstichig violett |
| 67 | 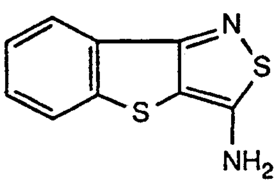 | 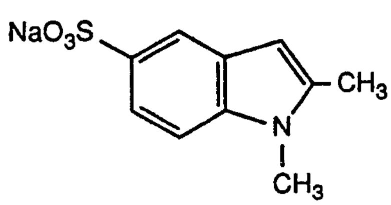 | bordeaux |
| 68 | 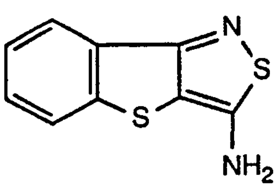 | 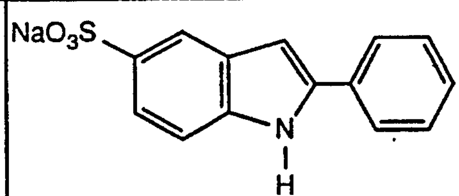 | rot |
| 69 | 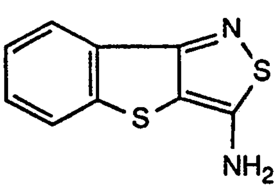 | 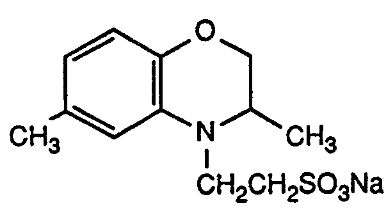 | blau |

| Beispiel | Diazokomponente | Kupplungskomponente | Nuance auf Polyamid |
|---|---|---|---|
| 70 | 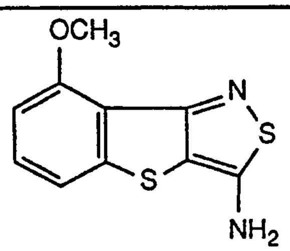 | 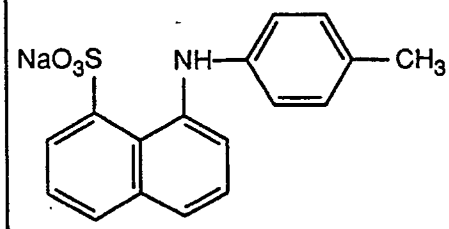 | grünstichig blau |
| 71 | 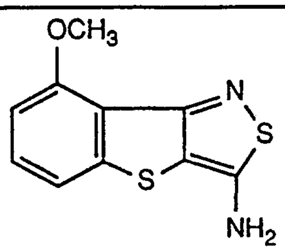 | 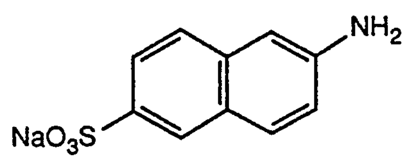 | rot |
| 72 | 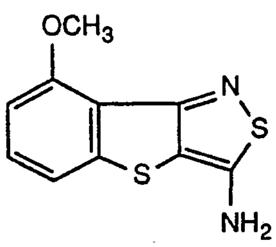 | 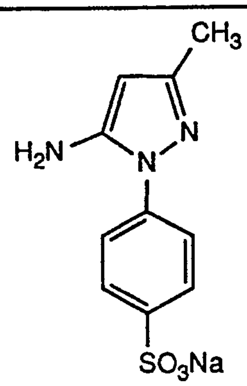 | orangegelb |
| 73 | 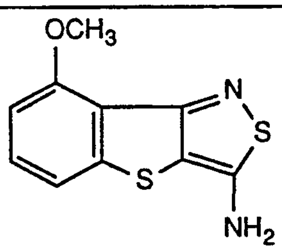 | 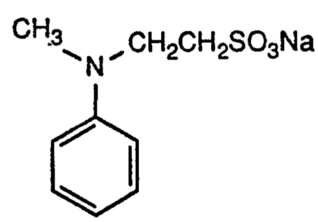 | violett |
| 74 | 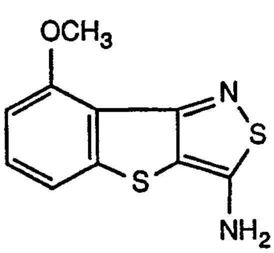 | 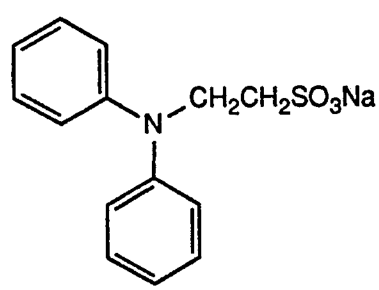 | rotviolett |

| Beispiel | Diazokomponente | Kupplungskomponente | Nuance auf Polyamid |
|---|---|---|---|
| 75 | 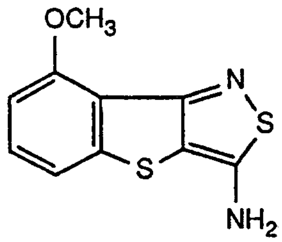 | 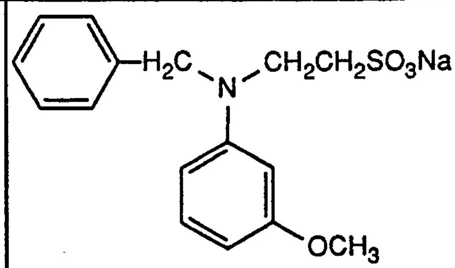 | rotstichig violett |
| 76 | 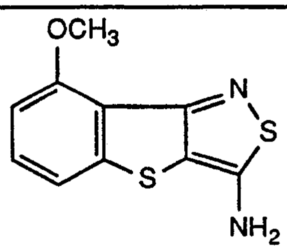 | 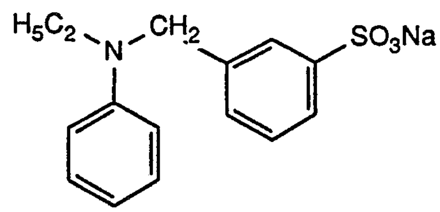 | violett |
| 77 | 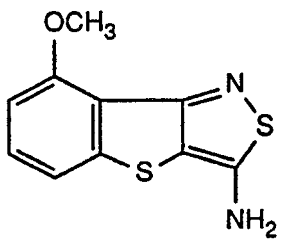 | 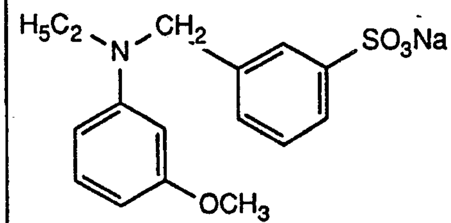 | rotstichig violett |
| 78 | 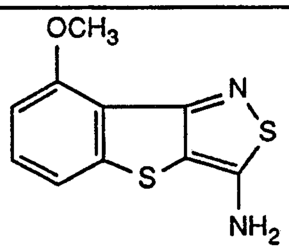 | 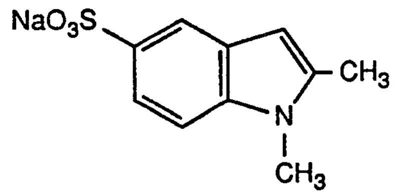 | bordeaux |
| 79 | 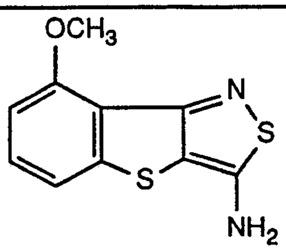 | 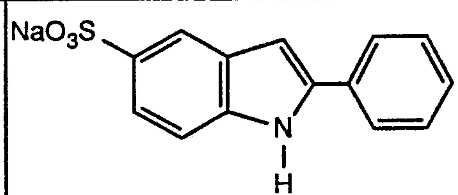 | rot |
| 80 | 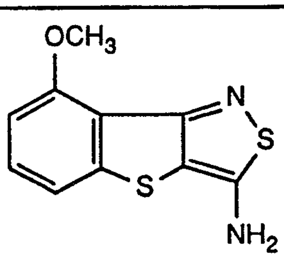 | 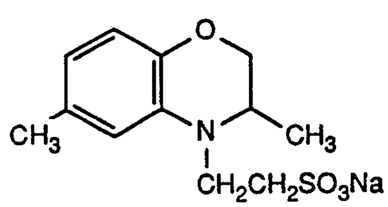 | blau |

| Beispiel | Diazokomponente | Kupplungskomponente | Nuance auf Polyamid |
|---|---|---|---|
| 81 | 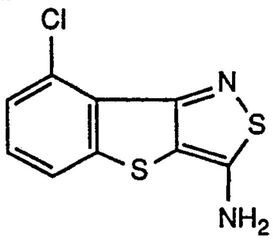 | 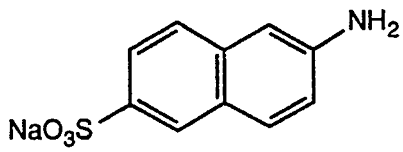 | rot |
| 82 | 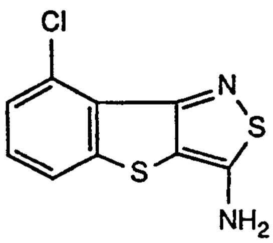 | 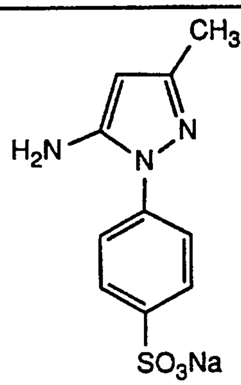 | orangegelb |
| 83 | 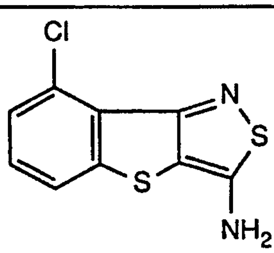 | 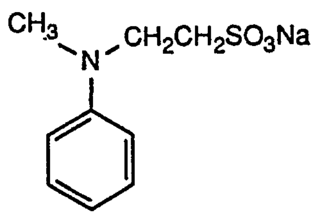 | violett |
| 84 | 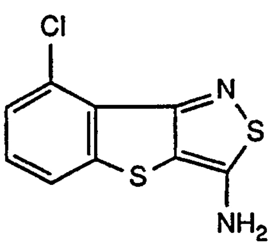 | 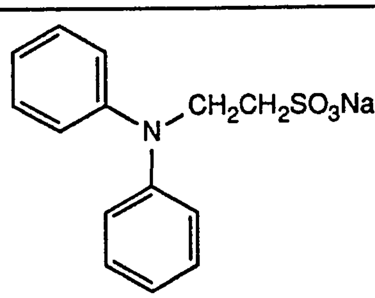 | rotviolett |

| Beispiel | Diazokomponente | Kupplungskomponente | Nuance auf Polyamid |
|---|---|---|---|
| 85 | 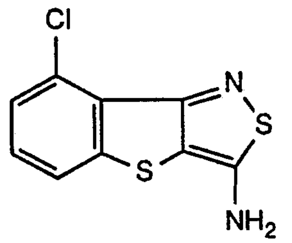 | 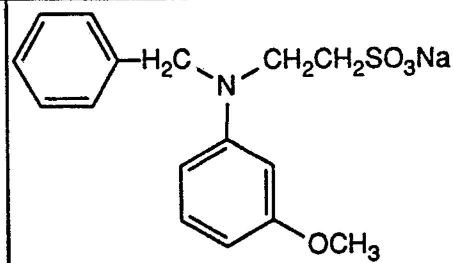 | rotstichig violett |
| 86 | 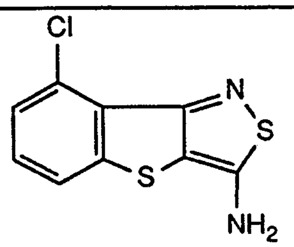 | 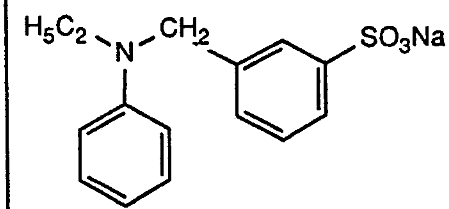 | violett |
| 87 | 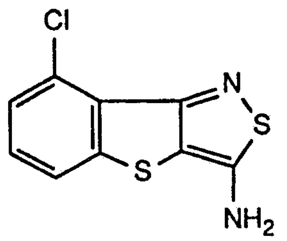 | 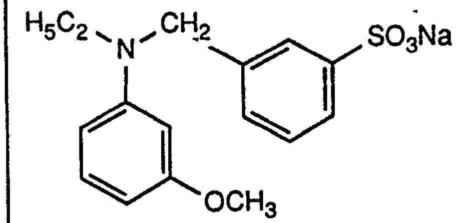 | rotstichig violett |
| 88 | 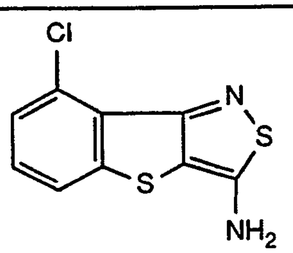 | 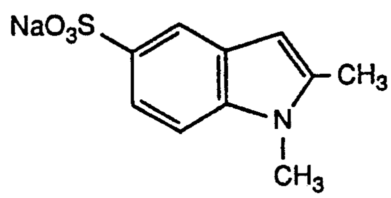 | bordeaux |
| 89 | 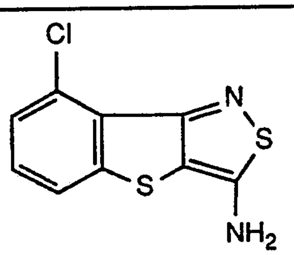 | 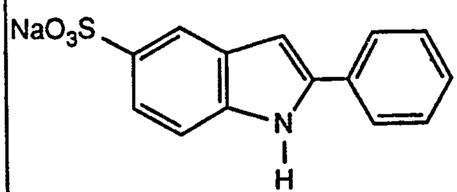 | rot |
| 90 | 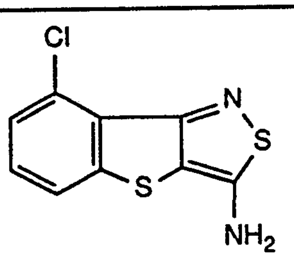 | 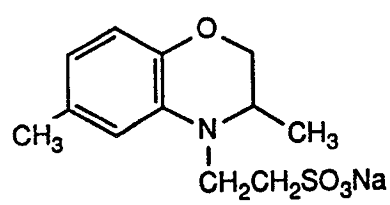 | blau |

Beispiel 91:

Man mischt 1 Teil des trockenen, coupagefreien Farbstoffs gemäss Beispiel 3 in einer Sandmühle zusammen mit 2 Teilen Dinaphthylmethandisulfonat (Na-Salz), 34 Teilen Quarzsand und 17 Teilen Wasser und mahlt das Gemisch so lange, bis eine Korngrösse von etwa 2 µm oder kleiner erreicht ist. Die entstehende Suspension wird vom Sand getrennt und der Sprühtrocknung unterworfen, wobei ein pulverförmiges Färbepräparat erhalten wird.

Beispiel 92:

Polyethylenglykolterephthalatgewebe wird auf einem Foulard bei 40°C mit einer Flotte folgender Zusammensetzung imprägniert:
20 Teile des gemäss Beispiel 91 erhaltenen Farbstoffpräparates fein dispergiert in
10 Teilen Natriumalginat,
20 Teilen Octylphenolpolyglykoläther und
930 Teilen Wasser.

Das auf ca. 60 % abgequetschte Gewebe wird bei 100°C getrocknet und anschliessend während 60 Sekunden bei einer Temperatur von 210°C fixiert. Die gefärbte Ware wird mit Wasser gespült, geseift oder reduktiv gereinigt und getrocknet. Man erhält eine lichtechte violette Färbung, welche sich vor allem durch eine gute Sublimierechtheit auszeichnet.

Beispiel 93:

2 Teile des gemäss Beispiel 91 erhaltenen Farbstoffpräparates werden in 4000 Teilen Wasser, enthaltend 12 Teile des Natriumsalzes von o-Phenylphenol, 2 Teile Ammoniumsulfat sowie 2 Teile des Natriumsalzes der Dinaphthylmethandisulfonsäure, dispergiert. Dann färbt man 100 Teile Garn aus Polyethylenglykolterephthalat 90 Minuten lang bei 95 bis 98°C in dieser Flotte.

Die Färbung wird anschliessend gespült und mit wässriger Natronlauge und einem Dispergator nachbehandelt. Man erhält so eine licht- und sublimierechte violette Färbung.

Beispiel 94:

1 Teil des gemäss Beispiel 4 erhaltenen Farbstoffs wird mit 2 Teilen einer 50%-igen wässerigen Lösung des Natriumsalzes der Dinaphthylmethandisulfonsäure nass vermahlen und getrocknet.

Dieses Farbstoffpräparat wird mit 40 Teilen einer 10%-igen wässerigen Lösung des Natriumsalzes der N-Benzylheptadecyl-benzimidazoldisulfonsäure verrührt und 4 Teile einer 40%-igen Essigsäurelösung zugegeben. Durch Verdünnen mit Wasser wird daraus ein Färbebad von 4000 Teilen bereitet.

In dieses Bad geht man bei 50°C mit 100 Teilen eines Polyesterfaserstoffes ein, steigert die Temperatur innert einer halben Stunde auf 120 bis 130°C und färbt eine Stunde in geschlossenem Gefäss bei dieser Temperatur. Anschliessend wird gut gespült. Man erhält eine blaustichig rote Färbung mit guten Echtheiten, insbesondere guter Sublimations- und Thermomigrationsechtheit.

Beispiel 95:

Man färbt 10 Teile Helancatricot (Polyamid) in 500 Teilen einer wässrigen Flotte, die 2 g/l Ammonacetat enthält und mit Essigsäure auf pH 5 gestellt wird. Die Flotte enthält 0,7 %, bezogen auf das Fasergewicht, des Farbstoffes gemäss Beispiel 8. Die Färbedauer bei einer Temperatur von 98°C beträgt 30 bis 90 Minuten. Das gefärbte Helancastück wird anschliessend herausgenommen und wie üblich gewaschen und getrocknet.

Man erhält ein brillant blau gefärbtes Helancastück, das eine reine Nuance und gute Gesamtechtheiten aufweist.

Beispiel 96:

Man färbt 10 Teile Helancatrikot (Polyamid) in 500 Teilen einer wässrigen Flotte, die 1 g/l Mononatriumphosphat enthält und mit Dinatriumphosphat auf pH 6 gestellt wird. Die Flotte enthält 1 % des Farbstoffes gemäss Beispiel 8, bezogen auf das Fasergewicht. Die Färbedauer bei einer Temperatur von 98 °C beträgt 30 bis 90 Minuten. Das gefärbte Helancastück wird anschliessend herausgenommen und wie üblich gewaschen und getrocknet.

Man erhält ein blau gefärbtes Helancastück, das eine reine Nuance und gute Gesamtechtheiten aufweist.

Beispiel 97:

Man färbte 10 Teile Wollstück in 500 Teilen einer wässrigen Flotte, enthaltend bezogen auf das Fasergewicht 0,45 % Farbstoff gemäss Beispiel 9, 5 % Glaubersalz kalz. und 4 % 96%-ige Schwefelsäure. Die Färbedauer bei einer Temperatur von 98 °C beträgt 30-60 Minuten. Das wie üblich gewaschene und getrocknete Wollstück weist eine rubinrote Färbung mit sehr guten Allgemeinechtheiten auf.

**Ansprüche**

1. Farbstoffe der Formel

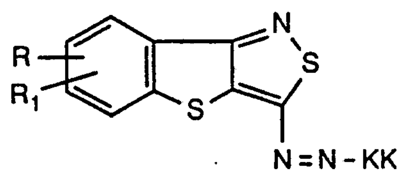

(1)

worin R und $R_1$ unabhängig voneinander je Wasserstoff, Halogen, Nitro, Alkyl oder Alkoxy und KK den Rest einer Kupplungskomponente bedeuten.

2. Farbstoffe gemäss Anspruch 1, worin R Wasserstoff, Chlor, Nitro, Methyl oder Methoxy und $R_1$ Wasserstoff ist.

3. Farbstoffe gemäss einem der Ansprüche 1 oder 2, worin KK den Rest einer Kupplungskomponente aus der Reihe der Acylacetarylamide, Phenole, Pyridone, Chinolone, Pyrazole, Indole, Diphenylamine, Aniline, Aminopyridine, Pyrimidine, Pyrimidone, Naphthole, Naphthylamine, Aminothiazole, Thiophene oder Hydroxypyridine bedeutet.

4. Farbstoffe gemäss Anspruch 3, worin KK den Rest einer Kupplungskomponente aus der Reihe der Acetoacetanilide, Phenole, Aniline, Diphenylamine, Naphthylamine, Naphthole, Indole, Chinoline, Pyridone, Pyrazole, Chinolone und Aminopyridine bedeutet, wobei diese Reste unsubstituiert oder durch Hydroxy, Amino, Alkylamino, Dialkylamino, Halogen, Alkoxy, Aryl, Aryloxy, Alkylcarbonylamino, Arylcarbonylamino, Alkylsulfonylamino oder Sulfo substituiert sind.

5. Farbstoffe gemäss Anspruch 4, worin KK den Rest eines Anilins, Naphthylamins oder Tetrahydrochinolins bedeutet, wobei diese Reste durch $C_1$-$C_4$-Alkyl, $C_1$-$C_4$-Alkylcarbonylamino, Phenyl, $C_1$-$C_4$-Alkylphenyl oder Sulfo substituiert sein können.

6. Farbstoffe gemäss Anspruch 1, worin $R_1$ Wasserstoff, R Wasserstoff, Chlor, Nitro, Methyl oder Methoxy und KK den Rest einer Kupplungskomponente aus der Reihe der Acetoacetanilide, Phenole, Aniline, Diphenylamine, Naphthylamine, Naphthole, Indole, Chinoline, Pyridone, Pyrazole, Chinolone und Aminopyridine bedeutet, wobei diese Reste unsubstituiert oder durch Hydroxy, Amino, Alkylamino, Dialkylamino, Halogen, Alkoxy, Aryl, Aryloxy, Alkylcarbonylamino, Arylcarbonylamino, Alkylsulfonylamino oder Sulfo substituiert sind.

7. Farbstoffe gemäss Anspruch 1, worin R und $R_1$ je Wasserstoff oder Chlor und KK den Rest eines Anilins, Naphthylamins oder Tetrahydrochinolins bedeutet, wobei diese Reste durch $C_1$-$C_4$-Alkyl, $C_1$-$C_4$-Alkylcarbonylamino, Phenyl, $C_1$-$C_4$-Alkylphenyl oder Sulfo substituiert sein können.

8. Verfahren zur Herstellung der Azofarbstoffe gemäss Anspruch 1, dadurch gekennzeichnet, dass man eine Verbindung der Formel

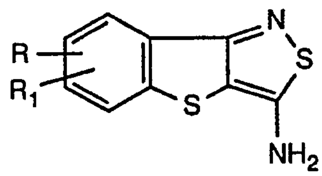

(2)

diazotiert und auf eine Kupplungskomponente

H-KK (3)

kuppelt, wobei R, $R_1$ und KK die unter der Formel (1) angegebene Bedeutung aufweisen.

9. Verbindungen der Formel

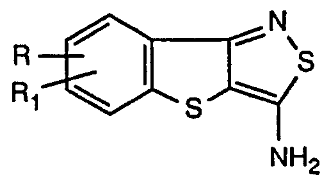

(2)

worin R und $R_1$ unabhängig voneinander Wasserstoff, Halogen, Nitro, Alkyl oder Alkoxy bedeuten.

10. Verfahren zur Herstellung der Verbindungen gemäss Anspruch 9, dadurch gekennzeichnet, dass man eine Verbindung der Formel

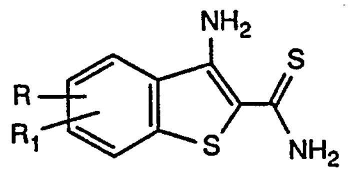

(4)

worin R und $R_1$ je Wasserstoff, Halogen, Nitro, Alkyl oder Alkoxy bedeutet, in Gegenwart einer organischen Base mit einem Oxidationsmittel behandelt.

11. Verbindungen der Formel

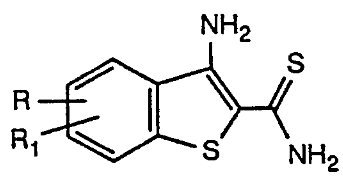

(4)

worin R und $R_1$ Wasserstoff, Halogen, Nitro, Alkyl oder Alkoxy bedeutet.

12. Verfahren zur Herstellung der Verbindungen gemäss Anspruch 11, dadurch gekennzeichnet, dass man in einer Verbindung der Formel

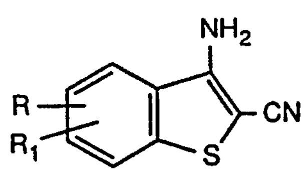

(5)

worin R und $R_1$ je Wasserstoff, Halogen, Nitro, Alkyl oder Alkoxy bedeutet, die Nitrilgruppe in die Thioamidgruppe umwandelt.

13. Verwendung der Azofarbstoffe gemäss Anspruch 1, welche keine wasserlöslichmachende Gruppe aufweisen, als Dispersionsfarbstoffe zum Färben oder Bedrucken von halbsynthetischen oder synthetischen hydrophobem Material, insbesondere Textilmaterial aus Polyesterfasern.

14. Verwendung der Azofarbstoffe gemäss Anspruch 1, welche mindestens eine Sulfogruppe aufweisen,

zum Färben und Bedrucken von Seide, Wolle und Polyamidmaterial, insbesondere von Textilmaterial aus Polyamidfasern.

15. Verfahren zum Färben oder Bedrucken von Textilmaterial, unter Verwendung einer oder mehrerer der im Anspruch 1 definierten Verbindungen.

16. Das gemäss Anspruch 15 gefärbte oder bedruckte Material.

European Patent Office

# EUROPEAN SEARCH REPORT

Application Number

EP 90 81 0123

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | GB-A-1 549 185 (ICI)<br>* Anspruch 1 *<br>--- | 1-8,13, 15,16 | C 09 B 29/039<br>C 07 D 333/70<br>C 07 D 513/04 //<br>D 06 P 1/16<br>D 06 P 1/39<br>(C 07 D 513/04<br>C 07 D 333:00<br>C 07 D 275:00 ) |
| Y | CHEMICAL ABSTRACTS, vol. 90, 1979, page 607, abstract no. 54894w, Columbus, Ohio, US; B. TORNETTA et al.: "Synthesis and spectral behaviour of pyridothienoisothiazole and pyridothienopyrimidine derivatives", & GAZZ. GHIM. ITAL. 1978, 108(1-2), 57-62<br>--- | 1-8,13, 15,16 | |
| X | IDEM<br>----- | 9-12 | |
| | | | TECHNICAL FIELDS SEARCHED (Int. Cl.5) |
| | | | C 09 B<br>C 07 D |

The present search report has been drawn up for all claims

| Place of search | Date of completion of the search | Examiner |
|---|---|---|
| THE HAGUE | 13-06-1990 | GINESTET M.E.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document